# EUROPEAN PATENT APPLICATION

(11) **EP 3 285 191 A1**
(43) Date of publication of application: **21.02.2018**
(21) Application number: 17185769.1
(22) Date of filing: 10.08.2017
(51) Int. Cl.: G06F 19/00

(54) **WORKSTATION, MEDICAL IMAGING APPARATUS, AND CONTROLLING METHOD THEREOF**

(30) Priority: 17.08.2016 KR 20160104375
(71) Applicant: Samsung Electronics Co., Ltd., Suwon-si, Gyeonggi-do (KR)
(72) Inventor: Hwang, Jin Young, Suwon-si, Gyeonggi-do (KR); Choi, Joon Sung, Anyang-si, Gyeonggi-do (KR)
(74) Representative: Gulde & Partner

(57) **Abstract**

A workstation, a medical imaging apparatus and a control method thereof are disclosed. In accordance with one aspect of the present disclosure, the workstation includes a display apparatus and a controller configured to receive respiration information of an object, determine an acquisition section of magnetic resonance (MR) data related to the object based on the respiration information. The controller is also configured to control the display apparatus to display the respiration information of the object and the acquisition section, and display a user interface configured to change the displayed acquisition section according to a user's operation command.

## Description

### BACKGROUND

### 1. Technical Field

Embodiments of the present disclosure relate to a work station for generating medical images based on respiration information of a target object, a medical imaging apparatus, and a control method thereof.

### 2. Description of the Related Art

In general, a medical imaging apparatus is a device that acquires a medical image by imaging the inside of a target object, and makes the acquired medical image available for diagnosis. Specifically, the medical imaging apparatus captures and processes structural details, internal tissue, and fluid flow within a body and presents them to a user.

A user such as a doctor may diagnose a health condition and a disease of a patient by using a medical image outputted from a medical imaging apparatus. As the accuracy of the medical image increases, a patient's condition may be more accurately judged. Accordingly, various methods for acquiring a medical image that minimizes an influence of a patient's condition have been studied as a method for acquiring clearer medical images.

### SUMMARY

Therefore, it is an aspect of the present disclosure to provide a workstation, a medical imaging apparatus and a controlling method of the medical imaging apparatus, which acquires a medical image with minimized artifacts caused by movement of an object.

In accordance with one aspect of the present disclosure, a workstation may include a display apparatus; and a controller configured to receive respiration information of an object, determine an acquisition section of magnetic resonance (MR) data related to the object based on the respiration information, and control the display apparatus to display the respiration information of the object and the acquisition section, and display a user interface configured to change the displayed acquisition section according to a user's operation command.

The controller may modify a protocol to correspond to the changed acquisition section when the displayed acquisition section is changed according to the user's operation command, and acquires MR data according to the modified protocol.

The controller may control the display apparatus to display the user interface implemented so that the displayed acquisition section and the changed acquisition section are provided together when the displayed acquisition section is changed according to the user's operation command.

The controller may control the display apparatus to display the user interface implemented so that the displayed acquisition section and the changed acquisition section are displayed differently when the displayed acquisition section is changed according to the user's operation command.

The controller may control the display apparatus to display the user interface implemented so that the displayed acquisition section and at least one of a color and a transparency of the changed acquisition section are set differently.

The controller may identify a change in an acquisition time and an acquisition length constituting the acquisition section when the displayed acquisition section is changed, and changes a parameter value related to a protocol according to the identification result.

The controller may control the display apparatus to display the user interface implemented to provide a message as to whether to modify a protocol according to the changed acquisition section when the displayed acquisition section is changed according to the user's operation command.

The controller may control the display apparatus to display the user interface implemented to display the acquisition section using at least one of an arrow, a line, a symbol, and a polygonal shape.

In accordance with another aspect of the present disclosure, a workstation may include a display apparatus; and a controller configured to receive respiration information of an object, determine a respiration state of the object, determine an acquisition section of magnetic resonance (MR) data based on the respiration information of the object and the respiration state of the object, and control the display apparatus to display the respiration information and the acquisition section of the MR data, and display a user interface implemented to receive a confirmation command indicating whether to set an imaging protocol to correspond to the displayed acquisition section.

The controller may set the protocol to correspond to the acquisition section when receiving a confirmation command from the user, and acquires MR data according to the set protocol.

The controller may control the display apparatus to display the user interface implemented to provide at least one of the acquisition section according to a predetermined protocol according to whether the protocol is pre-set and the acquisition section determined by the respiratory status

The controller may control the display apparatus to display the user interface implemented so that the acquisition section according to the predetermined protocol and at least one of a color and a transparency automatically determined according to the respiration status are set differently.

The controller may automatically determine the acquisition section using a respiration slope determined based on the respiration information and controls the display apparatus to display the user interface implemented to provide a message as to whether to set the protocol to correspond to the automatically determined acquisition section.

The controller may modify or set the protocol to correspond to any one of the acquisition section according to the predetermined protocol and the acquisition section determined according to the respiration status.

In accordance with the other aspect of the present disclosure, a controlling method of a medical imaging apparatus may include
receiving respiration information of an object; determining an acquisition section of magnetic resonance (MR) data related to the object based on the respiration information; displaying the respiration information of the object and the acquisition section of the MR data related to the object; displaying a user interface implemented on a display apparatus that permits a user to change the displayed acquisition section according to an operation command from the user; modifying an imaging protocol to correspond to the changed acquisition section when the displayed acquisition section is changed according to the operation command of the user, and acquiring MR data according to the modified imaging protocol.

The displaying may include displaying the user interface implemented on the display apparatus configured to provide the displayed acquisition section and the changed acquisition section together, when the displayed acquisition section is changed according to the user's operation command.

The controlling method of a medical imaging apparatus may further include determining a respiration state based on respiration information of an object; and determining an acquisition section of MR data according to the determined respiration state and displaying a user interface implemented on a display apparatus to receive confirmation of whether to set a protocol to correspond to the determined acquisition section.

The displaying may include displaying a user interface implemented on the display apparatus to provide at least one of the acquisition section according to the predetermined protocol and the acquisition section determined according to the respiration status.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a schematic diagram of an MRI system.
FIG. 2 is a schematic diagram illustrating a work space provided with a workstation.
FIG. 3 is a schematic diagram illustrating a process of generating image data according to an exemplary embodiment.
FIG. 4 is a schematic diagram illustrating the inside of a target object according to an embodiment.
FIG. 5 is a schematic diagram illustrating a change in position of a diaphragm based on respiration of an object according to an embodiment.
FIG. 6 a schematic diagram illustrating an acquisition section for each respiration cycle according to an embodiment.
FIG. 7 is a diagram illustrating a spirogram and an acquisition section for a respiration cycle according to an embodiment.
FIGS. 8A and 8B are diagrams illustrating that an acquisition section is changed based on a protocol according to an embodiment.
FIGS. 9 to FIG. 14 are diagrams illustrating screens displaying acquisition sections according to different embodiments.
FIG. 15 is a diagram illustrating a screen displaying an acquisition section and a message together.
FIGS. 16A and 16B are diagrams for explaining the determination of an acquisition section based on a respiration slope according to different embodiments
FIG. 17 is a diagram illustrating an acquisition section and a screen displaying a message.
FIG. 18 is diagram for explaining an operational flow of displaying a user interface on a display apparatus configured to support the display and change of an acquisition section based on respiration information.
FIG. 19 is a flowchart illustrating an operation flow for displaying a user interface configured to set an acquisition section using a respiration state based on respiration information and configured to display and confirm a set acquisition section.

### DETAILED DESCRIPTION

To clarify the scope of the present disclosure and enable one of ordinary skill in the art to which the present disclosure pertains to practice the present disclosure, the principles of the present disclosure are described and exemplary embodiments thereof are disclosed herein. The disclosed exemplary embodiments may be implemented in various forms.

Like reference numerals refer to like elements throughout. General information in the art to which the present disclosure pertains or overlapping information between embodiments will be omitted. The terms "part" and "portion" used herein may be implemented with software or hardware. A plurality of "parts" or "portions" may be implemented as a single unit or element according to embodiments, or a single "part" or "portion" may include a plurality of elements. Hereinafter, the principle of action and embodiments of the present disclosure will be described.

The image herein may include a medical image acquired by a medical imaging apparatus, such as a magnetic resonance imaging (MRI) apparatus, a computed tomography (CT) apparatus, an ultrasound imaging device, or an x-ray imaging device.

An "object" herein refers to an object to be captured and may include a human being, an animal, or a part thereof. For example, an object may include a part of a body (an organ), a phantom, or the like.

A magnetic resonance imaging (MRI) system acquires an magnetic resonance (MR) signal and restructures the acquired MR signal into an image. An MR signal refers to a radio frequency (RF) signal emitted from an object.

An MRI system has a main magnet forming a static magnetic field, and aligns a magnetic dipole moment direction of a particular atomic nucleus of an object placed in the static magnetic field to a direction of the static magnetic field. A gradient coil may apply a gradient signal to the static magnetic field, form a gradient magnetic field, and induce a resonance frequency to be different for each portion of the object.

An RF coil may radiate an RF signal corresponding to a resonance frequency of a portion desired for image acquisition. Also, as a gradient magnetic field is formed, the RF coil may receive MR signals having different resonance frequencies emitted from various portions of an object. By the above steps, the MRI system acquires an image from MR signals by using an image restoration technique.

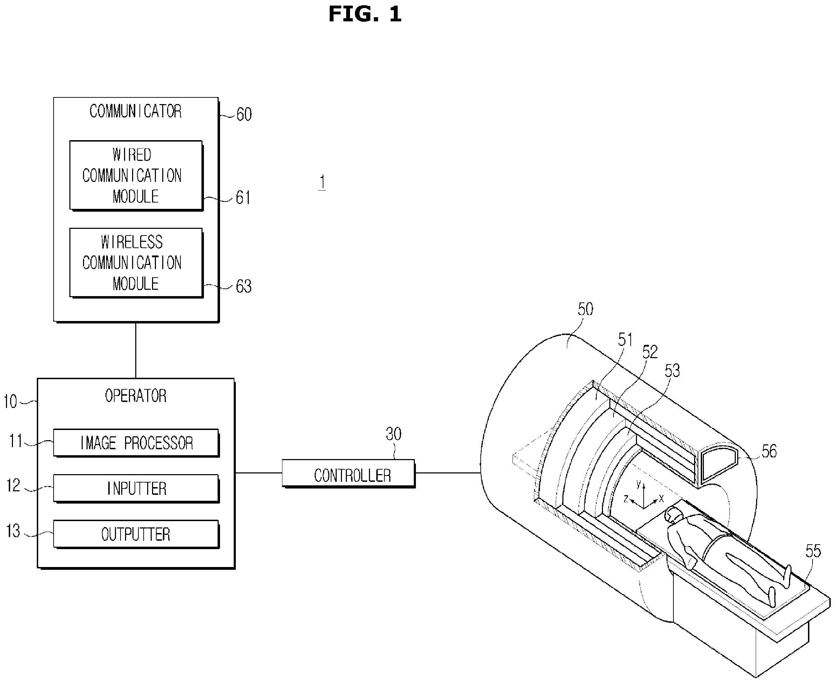

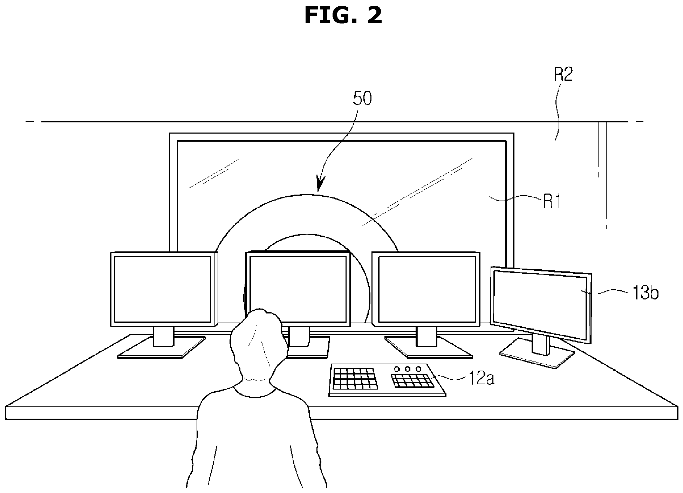

FIG. 1 is a schematic diagram of an MRI system and FIG. 2 is a schematic diagram illustrating a work space provided with a workstation. The embodiment will be described in connection with FIGS. 1 and 2 together to avoid overlapping explanation.

Referring to FIG. 1, a MRI system 1 may include an operator 10, a controller 30, and a scanner 50. Here, the controller 30 may be implemented independently as shown in FIG. 1. Alternatively, the controller 30 may be divided into a plurality of components and included in each component of the MRI system 1. Hereinafter, each component will be described in detail.

The scanner 50 may be embodied in a shape (for example, a bore shape) in which an internal space is empty and an object may be inserted. A static magnetic field and a gradient magnetic field are formed in the internal space of the scanner 50, and an RF signal is irradiated.

The scanner 50 may include a static magnetic field former 51, a gradient magnetic field former 52, an RF coil portion 53, a table 55 and a display 56. The static magnetic field former 51 forms a static magnetic field for aligning directions of magnetic dipole moments of atomic nuclei included in an object to a direction of the static magnetic field.. The static magnetic field former 51 may be implemented with a permanent magnet or may also be implemented with a superconducting magnet using a cooling coil.

The gradient magnetic field former 52 is connected to the controller 30. The gradient magnetic field former 52 applies a gradient to the static magnetic field according to a control signal received from the controller 30 and forms a gradient magnetic field. The gradient magnetic field former 52 includes an X-coil, a Y-coil, and a Z-coil forming a gradient magnetic field in an X-axis direction, a Y-axis direction, and a Z-axis direction perpendicular to one another, and generates a gradient signal corresponding to a capturing position to induce a resonance frequency to be different for each portion of the object. The RF coil portion 53 may be connected to the controller 30, radiate an RF signal to the object according to a control signal received from the controller 30, and receive an MR signal emitted from the object. The RF coil portion 53 may transmit an RF signal having a frequency identical to a precession frequency of an atomic nucleus performing the precession toward the object having the atomic nucleus, stop transmitting the RF signal, and receive an MR signal emitted from the object.

The RF coil portion 53 may be separately implemented as a transmission RF coil configured to generate an electromagnetic wave having an RF corresponding to a type of an atomic nucleus, and a reception RF coil configured to receive an electromagnetic wave emitted from the atomic nucleus. Alternatively, the RF coil portion 53 may also be implemented as a single RF transmission-reception coil having both transmitting and receiving functions. Also, a separate coil may be mounted to the object, in addition to the RF coil portion 53. For example, according to a capturing portion or a mounting portion, a head coil, a spine coil, a torso coil, a knee coil, or a type of coil specific to other body parts may be used as the separate coil.

The display 56 may be provided at the outside and/or the inside of the scanner 50. The display 56 may be controlled by the controller 30 and provide a user or an object with information related to medical imaging.

Also, the scanner 50 may include an object monitoring information acquirer configured to acquire and transmit monitoring information related to a state of an object. For example, the object monitoring information acquirer (not illustrated) may acquire monitoring information related to an object from a camera (not illustrated) configured to acquire the movement, position, and the like of the object, a spirometer (not illustrated) configured to measure the breath strength of the object, an electrocardiogram (ECG) meter (not illustrated) configured to measure the ECG of the object, or a thermometer (not illustrated) configured to measure the body temperature of the object, and transmit the acquired monitoring information to the controller 30. Accordingly, the controller 30 may use the monitoring information related to the object and control an operation of the scanner 50. Hereinafter, the controller 30 will be described.

The controller 30 may control an overall operation of the scanner 50.

The controller 30 may control a sequence of signals formed inside the scanner 50. The controller 30 may control the gradient magnetic field former 52 or the RF coil portion 53 according to a pulse sequence received from the operator 10 or a designed pulse sequence.

A pulse sequence includes all the information required to control the gradient magnetic field former 52 and the RF coil portion 53. For example, a pulse sequence may include information related to the intensity, duration, timing and the like of a pulse signal applied to the gradient magnetic field former 52.

The controller 30 may control the formation of a gradient magnetic field by the gradient magnetic field former 52 by controlling a waveform generator (not illustrated) configured to generate a gradient waveform, i.e., a current pulse, according to a pulse sequence and a gradient amplifier (not illustrated) configured to amplify a generated current pulse and transmit the amplified current pulse to the gradient magnetic field former 52.

The controller 30 may control the operation of the RF coil portion 53. For example, the controller 30 may supply an RF pulse of a resonance frequency to the RF coil portion 53 and control the RF coil portion 53 to radiate an RF signal, and receive an MR signal received by the RF coil portion 53. Here, the controller 30 may control the operation of a switch (e.g., a transmit/receive (T/R) switch) capable of controlling transmitting and receiving directions by a control signal and control RF signal radiation and MR signal reception according to an operation mode.

The controller 30 may control the movement of the table 55 on which an object is placed. Before capturing is performed, the controller 30 may move the table 55 in advance corresponding to a capturing portion of an object.

The controller 30 may control the display 56. For example, the controller 30 may control an on/off state of the display 56, a screen displayed on the display 56, or the like by using a control signal.

The controller 30 may be implemented with a memory or storage (not illustrated) configured to store an algorithm for controlling operations of elements within the MRI system 1 and data in a program form, and a processor (not illustrated) configured to perform the above-described operations by using the data stored in the memory. Here, the memory/storage and the processor may be implemented with separate chips. Alternatively, the memory/storage and the processor may also be implemented with a single chip.

The operator 10 may control the overall operation of the MRI system 1. The operator 10 may include an image processor 11, an inputter 12, and an outputter 13.

The image processor 11 may use the memory/storage to store an MR signal received from the controller 30 and use an image processor to apply an image restoration technique, thereby generating image data related to the object from the stored MR signal.

For example, when a k-space (also referred to as a Fourier space or a frequency space, for example) of the memory/storage is filled with digital data and k-space data is completed, the image processor 11 may apply various image restoration techniques with an image processor (e.g., take inverse Fourier transform of the k-space data) and restore the k-space data into image data.

Also, various types of signal processing applied to an MR signal by the image processor 11 may be performed in parallel. For example, a plurality of MR signals received by a multi-channel RF coil may be processed in parallel to be restored into image data. Meanwhile, the image processor 11 may store the restored image data in the memory/storage. Alternatively, as will be described below, the controller 30 may store the restored image data in an external server through a communicator 60.

The image data includes navigator image data and medical image data. Here, the navigator image data means image data used for tracking respiration of the object, and the medical image data means image data used for generating a magnetic resonance image of the object. The navigation data means an image reconstructed from digital data obtained in a training stage, and the medical image data means an image reconstructed from digital data acquired in an imaging stage. A detailed description thereof will be given later.

The inputter 12 may receive a control command related to the overall operation of the MRI system 1 from a user. For example, the inputter 12 may receive object information, parameter information, scan conditions, pulse sequence information, and the like from the user. The inputter 12 may be implemented with a keyboard, a mouse, a trackball, a voice recognizer, a gesture recognizer, a touchscreen, or the like, or combination of at least two of these components.

The outputter 13 may output image data generated by the image processor 11. Also, the outputter 13 may output a user interface (UI) configured so that the user may receive a control command related to the MRI system 1. The outputter 13 may be implemented with a speaker, a printer, a display, or the like, or a combination of these components.

Additionally, although the operator 10 and the controller 30 are illustrated as separate objects in FIG. 1, the operator 10 and the controller 30 may also be included in a single apparatus as described above. In addition, processes performed by each of the operator 10 and the controller 30 may be performed by other objects. For example, the image processor 11 may convert an MR signal received by the controller 30 into a digital signal, or the controller 30 may directly convert the received MR signal.

In one embodiment, at least one of the operator 10, and the controller 30 may be included in a workstation. Here, the workstation is referred to as a host device or a console, and will be referred to as a workstation for convenience of description in the above-described embodiments.

Some components of the MRI system 1 may be divided into a separate scan room where the object is scanned, and a control room that controls a scan of the object. Accordingly, a user may acquire the desired information through an image processing of an acquired medical image as well as a scan of the object in the control room.

However the workstation may not be provided only in the control room. For example the process of controlling scanning of the object may be provided in the control room, and the image processing may be provided in a separate room. At this time, the workstation provided in the control room corresponds to a first workstation, and the workstation provided in the separate room corresponds to a second workstation. The workstation described below includes at least one of the first workstation and the second workstation.

Meanwhile, the MRI system 1 includes the communicator 60 and may be connected via the communicator 60 to an external apparatus (not illustrated) (e.g., a server, a medical apparatus, a portable device (a smartphone, a tablet personal computer (PC), a wearable device, and the like).

The communicator 60 may include one or more elements that enable communication with an external apparatus, and may include, for example, one or more of a short-range communication module (not illustrated), a wired communication module 61, and a wireless communication module 62. The short-range communication module may be implemented with communication circuitry that is configured to communicate via Bluetooth, Wi-Fi, or another method. The wired communication module may be implemented with communication circuitry that is configured to communicate via Ethernet or another wired method. The wired communication module may be implemented with communication circuitry that is configured to communicate via Wi-Fi, LTE, or other communication methods.

The communicator 60 may receive a control signal and data from an external apparatus and transmit the received control signal to the controller 30 so that the controller 30 controls the MRI system 1 according to the received control signal.

Alternatively, the controller 30 may transmit a control signal via the communicator 60 to an external apparatus and control the external apparatus according to the control signal of the controller.

For example, the external apparatus may process data generated by the MRI system according to the control signal of the controller 30 received via the communicator 60.

A software program capable of controlling the MRI system 1 may be installed in the external apparatus, and the program may include instructions for performing some or all of the operations of the controller 30.

The software program may be installed in advance in the external apparatus, or may be downloaded from a server providing applications and installed by a user of the external apparatus. The server providing applications may include a recording medium having a corresponding program stored therein.

In addition to the above-described operations, the controller 30 may display a user interface on the display 56 that allows the user to intuitively grasp an acquisition section of MR data. Alternatively, the controller 30 may display the user interface on a separate electronic device such as a personal computer or a tablet. In addition, the controller 30 may perform various operations such as generating medical image data by controlling an operation of the components of the MRI system 1 according to a protocol. Hereinafter, a method for acquiring respiration information and setting an acquisition section in order to generate medical image data will be described in more detail.

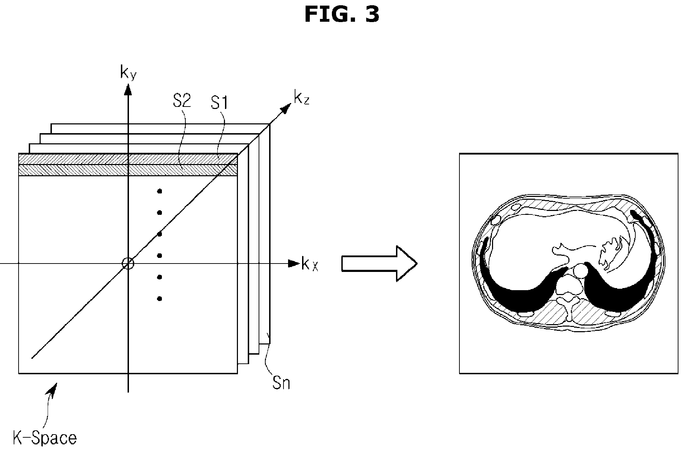

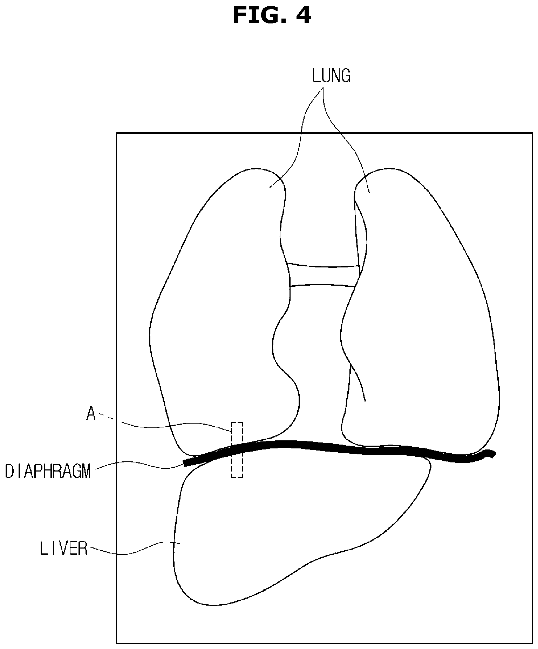

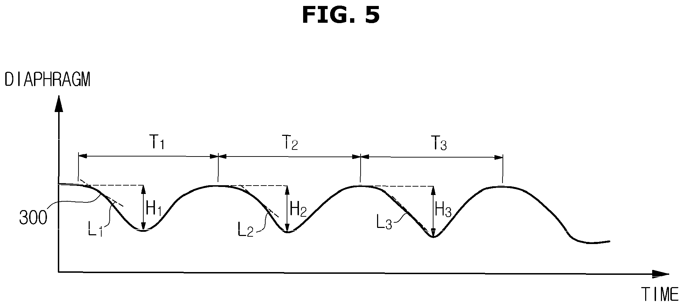

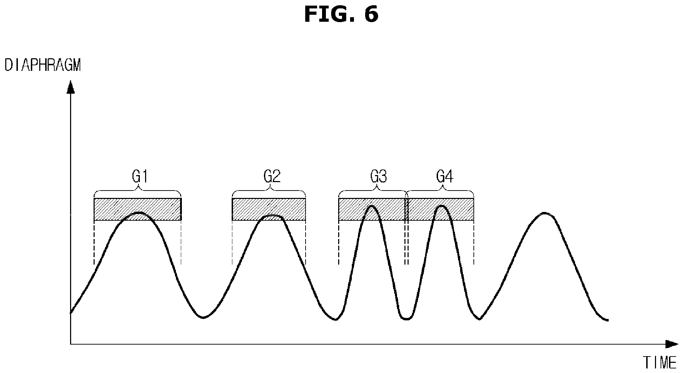

FIG. 3 is a schematic diagram illustrating a process of generating image data according to an exemplary embodiment and FIG. 4 is a schematic diagram illustrating the inside of a target object according to an embodiment. FIG. 5 is a schematic diagram illustrating a change in position of a diaphragm based on respiration of an object according to an embodiment, and FIG. 6 a schematic diagram illustrating an acquisition section for each respiration cycle according to an embodiment. The embodiment will be described together to avoid overlapping explanation.

At least one of the operator 10 and the controller 30 may be included in the workstation or included in another device. Therefore, the following description will be made on the assumption that the operator 10 and the controller 30 are included in the workstation, but the present invention is not limited thereto.

The controller 30 may perform various operations for acquiring the medical image data. For example, the controller 30 may collect a MR signal radiated from an object through the RF coil portion 53. In addition, the controller 30 may preprocess the collected MR signals. In one embodiment, the controller 30 may perform an operation of amplifying the MR signal. In addition, the controller 30 may convert an analog signal into an electrical signal, that is, output it as digital data. Hereinafter, MR data is referred to as MR data obtained by converting the MR signal to digital data.

The controller 30 may acquire the MR data while crossing a learning step and an imaging step. Hereinafter, navigator MR data is data obtained in the learning step and restored to the navigator image data, and medical MR data is data obtained at the imaging step and restored into medical image data. However, when there is no particular need to divide them separately, the navigator MR data and the medical MR data are unified into the MR data.

The MR signal received from the object may be reconstructed into image data. The image data includes navigator image data and a medical image data. Here, the navigator image data means image data used for tracking the respiration of the object, and medical image data means image data used for medical diagnosis of the object. When there is no particular need to divide them separately, the navigator image data and the medical image data may be unified into the image data.

The controller 30 may obtain respiration information of the object by tracking a movement of an organ generated by breathing, using the navigator image data. A detailed description thereof will be given later.

The controller 30 may collect the navigator MR data and the medical MR data at an intersection according to the protocol. In other words, the controller 30 may collect navigator MR data and medical MR data while crossing the learning step and the imaging step according to a set protocol. The protocol is a set of settings needed to acquire medical image data. In order to acquire the medical image data, the learning step and the imaging step may be performed in an intersecting manner. Accordingly, the controller 30 determines a respiration state of the object using the navigator image data acquired in the learning step based on the protocol, and minimizes artifacts of a restored medical image data, that is, noise, by using the detection result in the imaging step.

For example, the protocol may be set by a parameter associated with a setting of the acquisition section of the MR data. At this time, the acquisition section may be determined by at least one of an acquisition time and an acquisition length. Therefore, the acquisition section may be determined by parameters related to the acquisition time, and parameters related to acquisition length. Parameters related to the acquisition time and the acquisition length will be described later in detail.

Referring to FIG. 3, the controller 30 may fill the MR data on a k-space according to a protocol. The k-space may be filled with raw data for generating one image data, and the controller 30 may fill the k-space with raw data collected according to the protocol, that is, MR data.

Acquisition of image data may be performed in slice units. In other words, the amount of MR data acquired in the acquisition section for each respiration cycle may be set by the slice that is one of the parameters of the protocol. The slice values may be the same or different for each respiration cycle. For example, the sizes of a first slice S1 and a second slice S2 may be the same or different. The slice value may be set the same for every respiration cycle or differently for each respiration cycle.

When the k-space is filled with the slices S1, S2, and Sn, the controller 30 may transmit the MR data, that is, the k-space data, to the image processor 11. Then, the image processor 11 may restore the k-space data into the image data through the image restoration technique. Alternatively, the controller 30 may restore the k-space data to image data through an image restoration technique.

As the k-space is filled with the MR data received when a motion of the object is at a minimum, the controller 30 can generate clearer image data. For example, when the object is a living organism such as a person, an animal, or the like, the more the k-space is filled with the MR data received when the diaphragm movement is minimized, the more the noise of the reconstructed image data is minimized.

The controller 30 may sequentially acquire the MR data received from the object and fill in the k-space when exhaled. In one embodiment, the controller 30 may acquire MR data in units of slices S1, S2, and Sn as shown in FIG. 3. Since the slices S1, S2 to Sn filling the k-space are separated by phase and frequency encoding, the phase encoding gradient magnetic field and the frequency encoding gradient magnetic field may be applied differently for each slice.

The controller 30 may track the respiration of the object and acquire respiration information. At this time, the controller 30 may obtain respiration information using various devices capable of detecting respiration of the object.

For example, the controller 30 may obtain respiration information using a respiratory navigator that detects the respiration of the object. In one embodiment, the controller 30 may generate navigator image data for motion observation, and observe movement of the organ generated by breathing, and use the observation results to generate medical image data.

In addition, the controller 30 may acquire respiration information through various known devices, and is not limited to the above-described examples. Hereinafter, the respiration detection method of the controller 30 using the respiration detection navigator will be described as an example of the respiration detection method. However the embodiments to be described later are not limited thereto, but respiration information may be obtained through various known methods.

The controller 30 may perform the learning step and the imaging step in an alternate manner. Here, the learning step corresponds to observing a movement for setting or changing a protocol, and the imaging step corresponds to acquiring medical MR data on which the medical image data is based.

For example, the controller 30 may obtain respiration information using the navigator image data restored based on the navigator MR data. Here, the respiration information includes various information indicating a respiration state of the object. The respiration information may include at least one of a spirogram, a respiration cycle, a respiratory gradient, and a respiratory rate of the subject. In addition, the controller 30 may detect the respiration of the object and obtain respiration information including various parameters indicating the respiration state of the object.

Referring to FIG. 4, when the object exhales to vent air, the diaphragm rises toward the lungs. When the object inhales air, the diaphragm is directed toward the lungs. That is, the diaphragm moves up and down based on the respiration phase of the object. Since a position of the lung or a liver adjacent to the diaphragm also changes with the movement of the diaphragm, a movement of the diaphragm may be traced according to a movement of a boundary formed by the lung and the liver.

The controller 30 may track the respiration of the object by using the diaphragm movement by the respiration of the object. The controller 30 may track the movement of the diaphragm from the navigator image data. For example, the navigator image data may be image data relating to a region where the diaphragm is located in the organs of the object. At this time, the controller 30 may track the motion of the diaphragm using the navigation image data restored based on the MR data collected in the learning step.

In one embodiment, when the navigator image data for area A in FIG. 4 is displayed in time order, a graph as shown in FIG. 5 may be obtained. A boundary line included in the graph, that is, a spirogram 300, is located between the lung and the liver and corresponds to the movement of the diaphragm. In FIG. 5, a horizontal axis represents a time, and a vertical axis represents a position of the diaphragm.

In inspiration, the lung expands and the liver is contracted, so that the spirogram 300 between the lung and the liver moves downward. Therefore, when the spirogram 300 is lowered and located in the lower end region, it indicates when the object inhales. Conversely, when the object exhales, the lung is contracted and the lung expands, so that the spirogram 300 between the lung and the liver moves to the upper region. When the spirogram 300 is raised and located in the upper region, it indicates when the object exhales.

Noise generated in the image data may be minimized only if a protocol is set such that MR data is acquired when the movement of the object is at a minimum, that is, when the object exhales. The controller 30 may obtain respiration information from the spirogram 300 obtained by tracing the movement of the diaphragm to determine the respiration state. The respiration information is, for example, a respiration gradient (L1, L2, L3) with time, a respiration cycle (T1, T2, T3) with time, and a respiration height (H1, H2, H3). The controller 30 may determine the respiration state of the object based on the respiration information. On the other hand, the respiration cycle is also referred to as respiration length, and will be described below as a uniform respiration cycle for convenience of explanation.

The first respiration cycle T1 and the second respiration cycle T2 may be the same or different. The first respiration gradient L1 and the second respiration gradient L2 may be the same or different. The first respiration height (H1) and the second respiration height (H2) may be the same or different.

Therefore, an appropriate acquisition section, that is, the acquisition time and the acquisition length may be the same or different for each respiration cycle. Accordingly, the controller 30 may acquire clear image data if the protocol is set for each respiration cycle according to the respiration state of the object, or for the same plural respiration cycles.

For example, when the object is a person, an average respiration cycle of a person is between about 3 seconds and 4 seconds. However, not all people breathe according to the average respiration cycle and Individual respiratory characteristics may vary. In addition, because of internal factors such as emotional changes, external factors such as external environmental changes, respiratory conditions may change over time. Therefore, even if the protocol is set according to the average respiration cycle, the sharpness of the medical image data is not guaranteed to be high.

First to fourth acquisition sections G1 to G4 may be set for each respiration cycle as shown in FIG. 6. The third acquiring section G3 and the fourth acquiring section G4 may overlap if the respiration state of the object changes with time. Then, the MR data acquired in different respiration states (inspiration or expiration) are filled in the k-space together, and noise may be generated in the restored medical image data.

The controller 30 may obtain clear medical image data only by setting the protocol more accurately by reflecting the respiration information of the object. However, it is inconvenient for the user to check the respiration information of the object in detail and individually set the protocol for each respiration cycle.

The MRI system 1 according to the embodiment is configured to display the respiration information and the acquisition section of the MR data of the object in order to allow the user to more easily grasp the information, and the MRI system 1 may display a user interface on the display apparatus that allows a user to intuitively determine whether or not a change of the acquisition section is necessary.

In addition, the MRI system 1 may display a user interface on the display apparatus that allows a user to intuitively determine whether or not a change of the acquisition section of MR data is necessary. The MRI system 1 automatically determines the acquisition section of the MR data based on the respiration information and then displays the user interface implemented on the display apparatus so that the user may confirm whether or not to set the protocol according to the automatically determined acquisition section.

The user interface described below may be a graphical user interface that graphically implements a screen displayed on the display apparatus so that various information and command exchange operations between the user and the MRI system 1 may be performed more conveniently. Here, the display apparatus may be the outputter 13, but may include any device capable of visually displaying various information, such as an LCD display or a touchscreen display, and is not particularly limited.

For example, the graphical user interface visually displays respiration information on a screen displayed through a display apparatus. The graphic user interface may be implemented to display various identifiers such as icons, buttons, and the like that may more easily change the acquisition section of the MR data. In addition, icons and buttons for easily inputting various control commands from the user may be displayed on the graphic user interface. A detailed description thereof will be given later.

The acquisition section may be determined by parameters related to the acquisition section as described above. The acquisition section may be referred to as a gating window, but hereinafter, the acquisition section will be unified for convenience of explanation.

The parameters associated with the acquisition section may include parameters associated with the acquisition time and parameters associated with the acquisition section. For example, the parameters associated with the acquisition time may be an accept position and an accept window.

As another example, there may be a slice and Echo Train Length (ETL), parameter associated with the acquisition length. The ETL is one of the parameters for determining the acquisition length. As the ETL value increases, the acquisition length increases. As the slice value increases, the acquisition length increases. Hereinafter, the ETL will be unified for convenience of explanation. On the other hand, the parameters for determining the acquisition section are not limited to those described above, and the acquisition section may be determined by various known parameters.

The controller 30 displays an acquisition section according to a predetermined protocol, and may manually correct the protocol only when there is a change request. Alternatively, the controller 30 may automatically correct without any change request, and automatically determine an acquisition section even if the protocol is not set in advance, and set the protocol according to the determined acquisition section.

Hereinafter, a case where a protocol is manually corrected so as to correspond to a changed acquisition section according to a user's operation command will be described first.

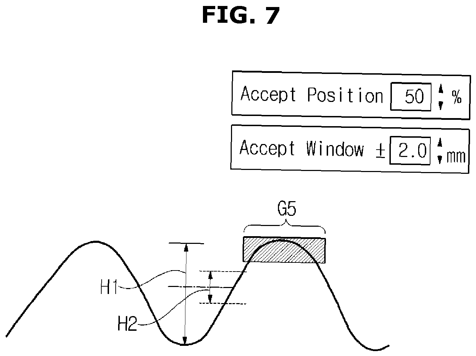

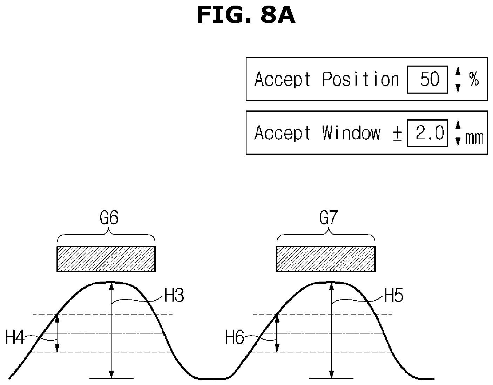

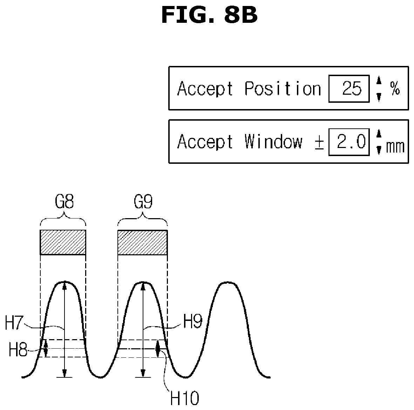

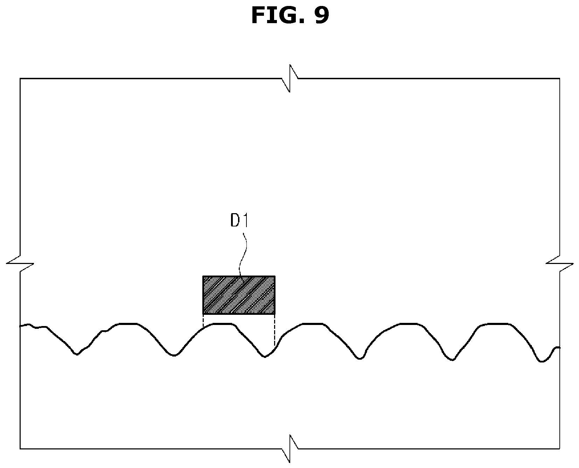

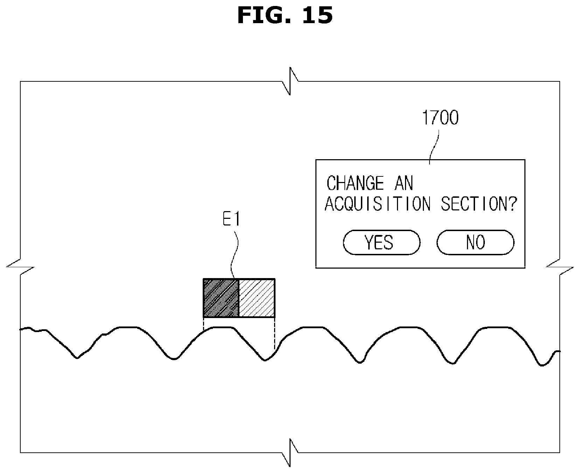

FIG. 7 is a diagram illustrating a spirogram and an acquisition section for a respiration cycle according to an embodiment. FIGS. 8A and 8B are diagrams illustrating that an acquisition section is changed based on a protocol according to an embodiment. FIG. 9, FIG. 10, FIG. 11, FIG. 12, FIG. 13, and FIG. 14 are a diagram illustrating screens displaying acquisition sections according to different embodiments, and FIG. 15 is a diagram illustrating a screen displaying an acquisition section and a message together. Hereinafter, the description will be given together to prevent duplication of description.

Referring to FIG. 7, the controller 30 may set the acquisition sections G5 based on the protocol-related parameters and the respiration information. At this time, an acceptance position may be set to 50%, and an acceptance window may be set to ± 2.0 mm. The above values may be preset by the user or pre-programmed into the MRI system 1.

The acceptance position means a parameter for determining the time of acquisition based on the respiration height. Further, the acceptance window means a parameter for determining the start range of the acquisition time point. For example, referring to FIG. 7, an acquisition start time may be determined within a range of ± 2.0 mm based on 50% of the respiration height (H1), that is, a half point.

The exact starting point of the acquisition may be pre-set through programs, algorithms, and so on. For example, the correct acquisition start time may be pre-set through a parameter value associated with the acquisition length, respiration information, a site to acquire image data, and the like.

In one embodiment, when the slice value and the ETL value exceed a predetermined value and the acquisition of a large amount of MR data is required within the acquisition section, the controller 30 determines a point of acquisition of 50% of the respiratory height H1, that is, a point of-2.0 mm based on the 1/2 point.

In another embodiment, when the slice value and the ETL value are less than the predetermined value, the controller 30 determines a point of acquisition of 50% of the respiratory height H1, that is, a point of +2.0 mm based on the 1/2 point. In addition, the acceptance window value may be set to any one of + 2.0 mm and -2.0 mm, and there is no limitation.

Respiration characteristics may vary from object to object. In addition, the respiratory characteristics may change over time due to internal factors and external factors as described above. For example, the spirogram shown in FIG. 8A may be confirmed to be steeper than the spirogram shown in FIG. 8B. In addition, it may be seen that the respiration cycle according to the spirogram of FIG. 8A is longer than the respiration cycle according to the spirogram of FIG. 8B.

When acquisition sections G8 and G9 of FIG. 8B are set to the same as acquisition sections G6 and G7 of FIG. 8A, the MR data at the time of inspiration and expiration are filled together on one k-space. Therefore, as shown in FIG. 8B, the acceptance position value should be changed to 25%.

In addition, since the respiration cycle is shorter, the acquisition length of the acquisition sections G8 and G9 in FIG. 8B should be shorter than the acquisition length of the acquisition sections G6 and G7 in FIG. 8A. Therefore, clear image data may be obtained only by changing the parameter value associated with the acquisition length.

When the object breathes as in the spirogram shown in FIG. 8A and comes to breathe as in the spirogram shown in FIG. 8B, both the parameter value related to the acquisition time and the parameter value related to the acquisition section should be changed.

More specifically, the acquisition time points of the acquisition sections G8 and G9 shown in FIG. 8B should be set faster than the acquisition sections G6 and G7 shown in FIG. 8A, and the acquisition sections G8 and G9 shown in FIG. 8A should be set shorter than the acquisition length of the acquisition sections G6 and G7 shown in FIG. 8A, so that vivid image data can be obtained. In addition, because the object's respiratory heights (H3, H5, H7, H9) may be the same or different, the respiratory height should also be considered in setting the parameters.

However, if the user individually sets the above-mentioned at least one parameter value every respiration cycle, inconvenience results. Therefore, the controller 30 according to the embodiment not only visually displays the respiration information and the acquisition section, but also displays the user interface implemented on the display apparatus so as to intuitively change the displayed acquisition section, thereby allowing the user to more intuitively grasp and change the acquisition section. In addition, the controller 30 according to the embodiment may modify at least one parameter value to correspond to the changed acquisition section.

In one embodiment, the controller 30 displays not only the acquired respiration information and the acquisition section of the MR data, but also a user interface implemented on the display apparatus so as to change the acquisition section more intuitively. The acquisition section may be set according to the parameter value and the respiration information as described above. At this time, the acquisition section may be expressed through various expression methods.

The acquisition section may be expressed using various known identifiers such as polygons, lines, arrows, and symbols, etc., so that the user identifies the acquisition section from various information displayed on the display apparatus.

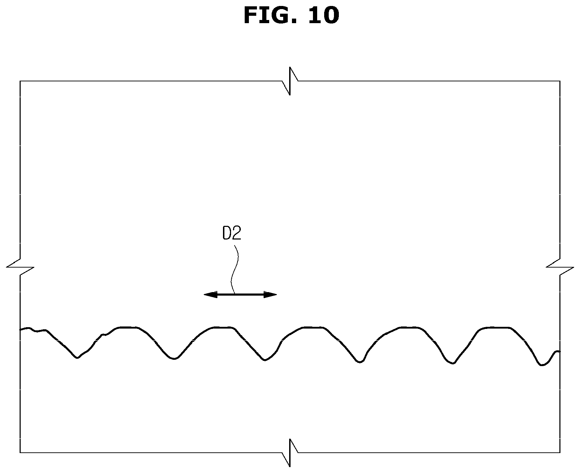

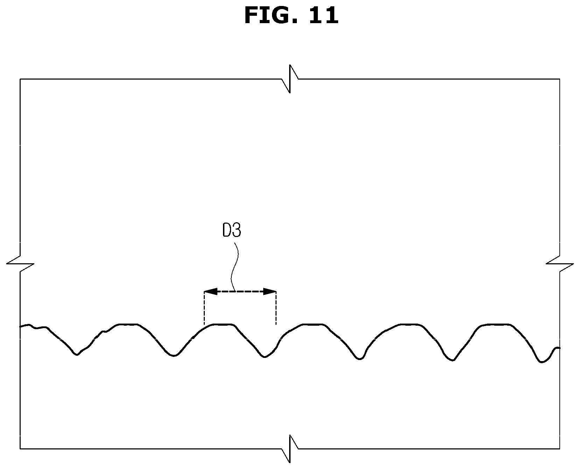

For example, the controller 30 may display a user interface implemented on the display apparatus to display an acquisition section D1 in the form of a rectangle as shown in FIG. 9. As another example, the controller 30 may display a user interface implemented on the display apparatus as shown by solid lines and arrows in an acquisition section D2 as shown in FIG. 10 As another example, the controller 30 may display on the display a user interface implemented apparatus as shown by a dotted line and arrows in the acquisition section D3 as shown in FIG. 11.

In addition, the user interface may be implemented so that the color, transparency, and the like representing the acquisition section can be freely adjusted. In addition, the acquisition section may be represented by other types of visual indicators, and is not limited to the above-described.

The user may check the acquisition section displayed on the display apparatus and change the acquisition section.

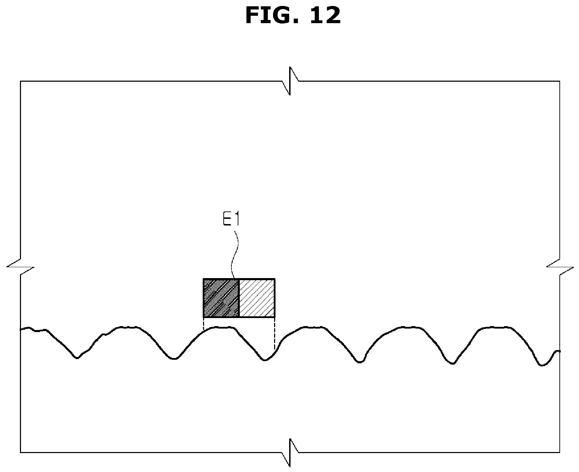

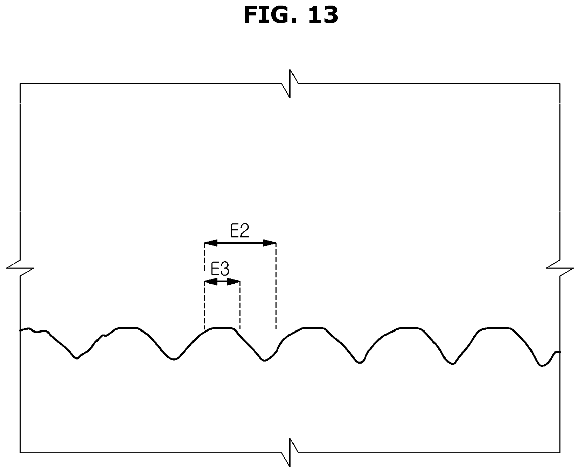

For example, when the display apparatus is implemented as a touch screen type, the controller 30 may change the acquisition section according to the touch operation of the user. The user may touch an acquisition section E1 shown in FIG. 12 and then change the acquisition section through a drag operation. At this time, the area indicated in FIG. 12 corresponds to the acquisition section changed by the user. In one embodiment, the user may change the acquisition time through the touch-drag and change only the acquired length as shown in FIG. 13.

Alternatively, upon receiving an operation command from a user through the inputter 12 implemented by a mouse, a keyboard, or the like, the controller 30 may change the acquisition section to correspond to the operation command, and display the changed acquisition section on the display apparatus.

The controller 30 may separately display the acquisition section E2 according to the predetermined protocol and an acquisition section E3 changed according to the user's operation command as shown in FIG. 13. At this time, as shown in FIG. 13, the controller 30 may display the acquisition section E2 according to the predetermined protocol and the acquisition section E3 changed according to the user's operation command with the same line and arrows.

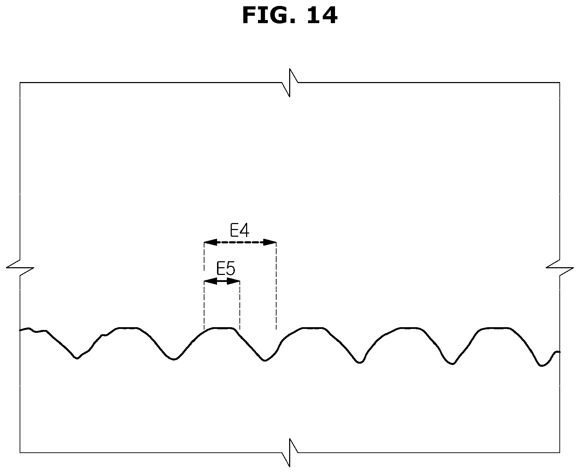

In addition, the controller 30 may display a user interface implemented on the display apparatus so as to differently display the acquisition section according to a predetermined protocol and the acquisition section changed according to a user's operation command. For example, as shown in FIG. 14, the user interface may be implemented so that the acquisition section E4 according to a predetermined protocol is indicated by a dotted line and an acquisition section E5 changed according to a user's operation command is indicated by a solid line, and there is no restriction on the display method.

In addition, the controller 30 may freely adjusts the color, transparency, and the like, and displays the user interface implemented on the display apparatus so as to be more easily distinguished between the acquisition section according to the predetermined protocol and the acquisition section changed according to the user's operation command. For example, referring to FIG. 12, an area indicated in bold in the acquisition section E1 corresponds to an acquisition section changed according to an operation command of the user, and the entire area including the highlighted section and the blurred section corresponds to the acquisition section according to the predetermined protocol.

The controller 30 may reset or modify the protocol to correspond to the changed acquisition section. For example, the controller 30 determines whether at least one of the acquisition time and the acquisition length constituting the acquisition section has been changed, and may modify at least one of the parameter values related to the acquisition time and the parameter value related to the acquisition length according to the determined result.

In one embodiment, when only the acquisition length is changed as shown in FIG. 12, the controller 30 may modify at least one of the slice value and the ETL value to be smaller. At this time, the method of determining which of the slice value and the ETL value is to be modified may be implemented in an algorithm or a program form and the method is stored in a memory of the controller 30 or may be stored in an external device. Alternatively, whether or not to modify any of the slice value and the ETL value may be selected by the user and is not limited.

A main controller 35 may collect the MR data and fill the k-space with respect to the acquisition section according to the changed protocol. The MRI system 1 according to the embodiment may decrease management time and improve convenience by allowing the user to reset or modify the protocol for each respiration cycle by a simple operation command without individually inputting the parameter values related to the protocol.

In particular, the imaging time may be the same or different for each inside area of the object to which the medical image data is to be acquired. When photographing an inside area requiring a long photographing time, there is a greater possibility that the respiration of the object changes instantaneously. Therefore the higher the possibility that the respiration is changed, the more the MRI system 1 according to the embodiment may reduce the time required for the user to modify the protocol.

Meanwhile, the method of changing the parameter value of the protocol to correspond to the changed acquisition section may be implemented in an algorithm or a program form and stored in the memory of the controller 30. Alternatively when the method is stored in an external device such as a server, the controller 30 may interface with an external device through the communicator 60 and change the parameter values of the protocol to correspond to the changed acquisition section.

The controller 30 may reset the protocol according to the changed acquisition section according to the user's operation command. Alternatively, the controller 30 may receive confirmation as to whether or not to reset the protocol according to the changed acquisition section, and then reset the protocol after receiving confirmation.

For example, the controller 30 may receive confirmation as to whether or not the protocol is reset by receiving a preset operation command. In one embodiment, when the user double-clicks with the mouse or presses a specific key via the keyboard, the controller 30 may reset the protocol according to the changed acquisition section. Alternatively, the controller 30 may display a user interface including an icon for receiving a confirmation command of the user on the display apparatus. Accordingly, when the user clicks the icon via the mouse, the protocol may be reset according to the changed acquisition section.

In another embodiment, the controller 30 may display a pop-up message 1700 on the display apparatus as shown in FIG. 15 so that the user may be prompted to modify the protocol according to the changed acquisition section.

As described above, the controller 30 may automatically correct the acquisition section even if there is no change instruction from the user, automatically determine the acquisition section even if the protocol is not set in advance, and set the protocol according to the determined acquisition section. Hereinafter, a method of performing the automatic correction or automatically determining the acquisition section will be described.

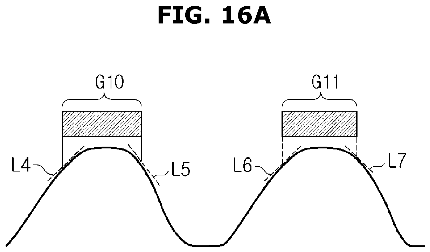

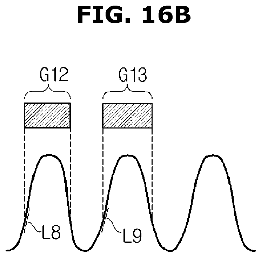

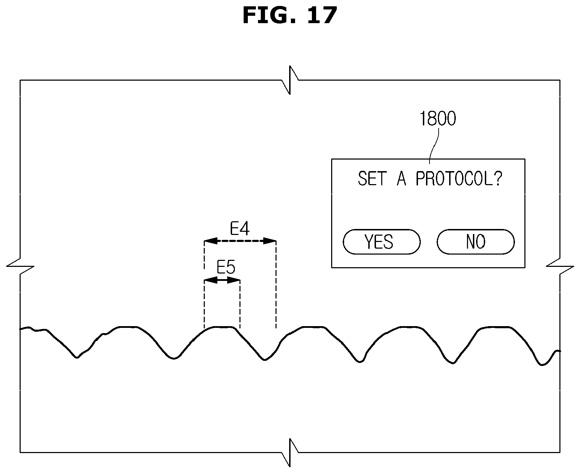

FIGS. 16A and 16B are diagrams for explaining the determination of an acquisition section based on a respiration slope according to different embodiments, and FIG. 17 is a diagram illustrating an acquisition section and a screen displaying a message.

The controller 30 determines the acquisition section to correspond to the respiration information, and may display a user interface, configured to display the determined acquisition section and receive confirmation from the user, on the display apparatus.

As described above, since the respiration state may be different for each object, clear medical image data may not be obtained when the protocol is set according to the average respiration state. However, there is a problem because it is too troublesome to individually set parameters related to the protocol in accordance with the respiration state every respiration cycle.

Accordingly, the controller 30 may determine the respiration state based on the respiration information, and determine the acquisition section according to the obtained result. In addition, the controller 30 may display a user interface on the display apparatus to provide the acquired acquisition section together with the respiration information, so that the user may be confirmed whether or not to reset the protocol according to the changed acquisition section.

At this time, the acquisition section may be determined for each respiration cycle. When the spirogram for each respiration cycle is similar to a preset range, the acquisition section may be determined to be the same for a similar respiration cycle within a predetermined range. Accordingly, the controller 30 may automatically determine the acquisition section to increase the convenience of the user, as well as to prevent an overload due to the increase in the amount of calculation.

The controller 30 may determine the acquisition section using the respiration slope from among various parameters that may determine the respiration state.

In one embodiment, the controller 30 may determine the acquisition section when the respiration slope of the spirogram is within a preset range. Referring to FIG. 5, in general, the respiration slope of the spirogram when exhaling corresponds to a certain range based on the time axis. Therefore, the controller 30 may determine the acquisition section for the region having the respiration slope of the spirogram corresponding to the predetermined range. As a specific example, the controller 30 may determine an acquisition section in which the respiratory gradient is -N ≤ respiration slope ≤ + M (N, M ≥ 0) based on the time axis.

In another embodiment, as referring to FIG. 16A the controller 30 may determine a point at which the slope of the spirogram corresponds to a predetermined slope L4 as the acquisition start time, or a point at which the slope of the spirogram becomes equal to or greater than the predetermined slope L4 as the acquisition start time. In addition, the controller 30 may determine a point at which the slope of the spirogram corresponds to a predetermined slope L5 as the acquisition end time, or a point at which the slope of the spirogram becomes equal to or less than a predetermined slope L5 as the acquisition end time. Then, the controller 30 may determine the acquisition section G10 between the acquisition start time and the acquisition end time.

In another embodiment, the controller 30 determines the slope L6 of the spirogram to be the predetermined acquisition start point, and the acquisition section from the acquisition start point to the point where the predetermined acquisition length has elapsed. At this time, the memory of the controller 30 or the external device stores data on the acquired length according to the slope of the spirogram, and the controller 30 may determine the acquisition section using the above-described data. In addition, the acquisition length may be determined not only by the slope of the spirogram, but also by various parameters indicating the respiration state, the type of object, and the internal area to be diagnosed. Alternatively, the acquisition length may be determined by the user.

The controller 30 may determine a point at which a slope of the spirogram corresponds to a predetermined slope or a point higher than a predetermined point as the acquisition start point. For example, as referring to FIG. 8B, when the respiratory slope of the specific point in the spirogram of the object is higher than the predetermined slope of the respiratory slope at the same point in the mean spirogram, that is the respiration is determined to be steep, the controller 30 may determine the specific point as the acquisition start point.

In one embodiment, as referring to FIG. 16B, when the slope of the spirogram is equal to predetermined slopes L8 and L9, the controller 30 may determine a point corresponding to the predetermined slopes L8 and L9 as the acquisition time point. At this time, the controller 30 may determine the acquisition section according to the acquisition time and the predetermined acquisition length. Here, the method in which the acquisition length is previously set is the same as that described above, so a detailed description thereof will be omitted.

When the parameter value of the protocol is set in advance, the controller 30 displays a user interface configured to provide an acquisition section according to a predetermined parameter value, an acquisition section automatically determined through the above method, and respiration information together, on the display apparatus. Accordingly, the controller 30 may determine whether to reset the parameter values of the protocol according to the acquisition section determined according to whether or not a predetermined operation command is input from the user.

For example, when the user double-clicks through the mouse or presses a specific key via the keyboard, the controller 30 may reset the parameter values of the protocol according to the automatically determined acquisition section. Alternatively, the controller 30 may display a pop-up message 1800 (FIG. 17) asking whether to reset the acquisition section E4 according to a predetermined protocol, the automatically determined acquisition section E5 and the parameter value of the protocol, and request the user for an active response. When the parameter value of the protocol is not set in advance, the controller 30 may display a user interface on the display apparatus configured to provide the acquisition section and the respiration information automatically determined through the above-described method. Accordingly, the controller 30 may determine whether to reset the parameter values of the protocol in accordance with the acquisition section, which is automatically determined based on whether or not a predetermined operation command is input from the user. Since the method of receiving the operation command is the same as described above, a detailed description thereof will be omitted.

On the other hand, the memory of the controller 30 may store data on the average respiration state for each object and data on the protocol setting based on the average respiration state. Accordingly, the controller 30 may determine the respiration state of the object, compare the obtained result with the data on the average respiration state, and then determine whether a specific situation occurs or not based on whether or not it is out of a preset range.

When it is determined that a specific situation has occurred, the controller 30 selects various methods of determining the acquisition section. The methods for determining the acquisition section includes a method of setting the acquisition section based on the average respiration state using the slope of the spirogram, and a method of automatically determining the acquisition section using the respiration state.

The above-described method of implementing a user interface may be implemented by an algorithm or a program, and be stored in the memory of the controller 30. The controller 30 may display the user interface on the display apparatus using the data stored in the memory.

Alternatively, the algorithms or programs described above may be stored in an external device. The controller 30 may receive data about a user interface derived from the algorithm or program transmitted from the external device through the communicator 60 and may display the received data on the display apparatus. The controller 30 may update data in cooperation with an external device through the communicator 60, and the data includes not only the above-described algorithm or program but also data relating to the average respiratory cycle, and data relating to the protocol setting method.

Hereinafter, the operation flow of the MRI system 1 for modifying the protocol according to whether or not the displayed acquisition section is changed after displaying the acquisition section will be described.

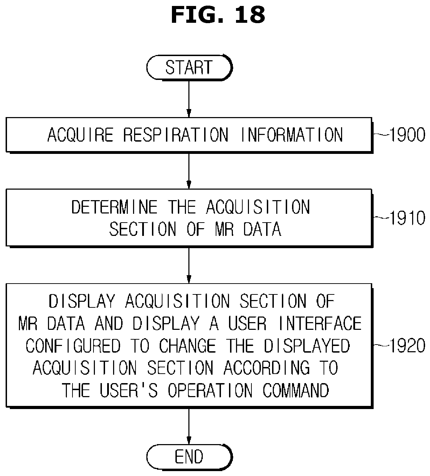

FIG. 18 is diagram for explaining an operational flow of displaying a user interface on a display apparatus configured to support the display and change of an acquisition section based on respiration information.

The MRI system 1 may acquire the MR signal only in a specific respiration state while monitoring the respiration state of a patient in real time at the same time as the scan. The MRI system 1 may acquire respiration information (1900). For example, the MRI system may acquire respiration information of an object using a respiration detection navigator. At this time, the respiration information is various information for understanding the respiration of the object, and may include various information such as a spirogram, a respiration cycle, and a spirogram slope.

The MRI system 1 may determine the acquisition section according to the respiration information and the predetermined protocol (1910). At this time, the acquisition section is an area for collecting a medical MR signal, and the medical MR signal is converted into the medical MR data through the signal processing, and is filled in one k-space.

The MRI system 1 may display a user interface on the display apparatus capable of changing the acquisition section so that a user can intuitively understand the respiration state and the acquisition section according to the predetermined protocol (1920).

For example, the MRI system 1 may display a user interface on the display apparatus to provide respiration information, and acquisition section according to a predetermined protocol. In particular the MRI system 1 may display a user interface on the display apparatus to provide a spirogram and an acquisition section D1 according to a predetermined protocol, as shown in FIG. 9.

The MRI system 1 may display a user interface on the display apparatus, the acquisition section of which is changed according to a simple operation command of the user. For example, the MRI system may provide the user interface in which an acquisition section is adjusted through a touch or touchscreen operation of the user. Alternatively, the MRI system 1 may provide the user interface with which the acquisition section may be adjusted according to a user's operation command using a mouse, a keyboard, or the like. Accordingly, the user may determine whether the acquisition section needs to be changed by comparing the acquisition section displayed on the display apparatus with the respiration information, and the acquisition section may be changed through the inputter 12.

When the acquisition section is changed, the MRI system 1 may collect the medical MR data in the changed acquisition section to fill a k-space image, and generate the medical image data using the data filled in the k-space. Alternatively, the MRI system 1 may provide the user interface that can be used to collect medical MR data in an acquisition section according to a predetermined protocol, or to confirm whether to collect medical MR data according to a changed acquisition section.

At this time, the MRI system 1 may display on the display the user interface apparatus to provide the acquiring section according to a predetermined protocol and the modified acquisition section together so that the user may analyze the sections more easily, side-by-side. Accordingly, the MRI system 1 according to the embodiment may permit the user to more accurately determine the acquisition section, thereby making it possible to generate medical image data that is more clear.

Hereinafter, an operation flow of an MRI system that automatically determines an acquisition section according to a respiration state, or provides a determined acquisition section according to a predetermined protocol and a respiration status will be briefly described.

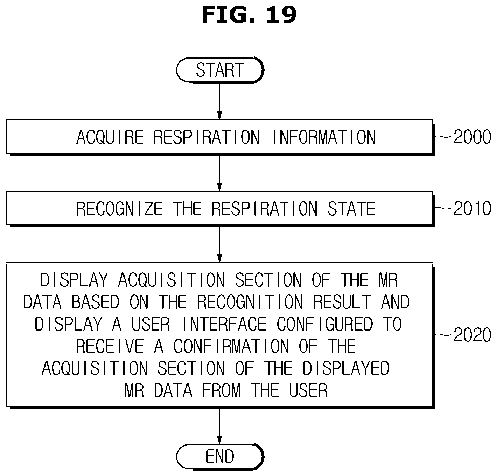

FIG. 19 is a flowchart illustrating an operation flow for displaying a user interface configured to set an acquisition section using a respiration state based on respiration information and configured to display and confirm a set acquisition section.

Referring to FIG. 19, the MRI system 1 may acquire respiration information (2000). The operation of acquiring the respiration information is the same as that of step 1900 described above, so a detailed description thereof will be omitted.

The MRI system 1 may recognize the respiration state using the obtained respiration information (2010), and determine the acquisition section of the MR data based on the recognition result. In addition, the MRI system 1 may display a user interface on the display configured to display the determined acquisition section and receive a confirmation of the acquisition section of the displayed MR data from the user (2020).

The MRI system 1 automatically determines the acquisition section of the MR data according to the respiration state, regardless of whether or not the parameter values relating to the protocol are pre-set. For example, the MRI system 1 may determine an acquisition section using at least one of a point at which the respiration slope becomes equal to or greater than a predetermined first slope and a point at which the respiration slope becomes equal to or less than a second predetermined slope. That is, the MRI system 1 may determine the acquisition section using at least one of the acquisition start time and the acquisition end time.

In one embodiment, the MRI system 1 may determine a point at which the breathing gradient becomes equal to or greater than a predetermined first slope as the acquisition start point, and a point at which the breathing gradient becomes equal to or less than the predetermined second slope as the acquisition end point. Accordingly, the MRI system 1 may determine the section between the acquisition start time and the acquisition end time as the acquisition section.

In another embodiment, the MRI system 1 may determine a point at which the respiration slope becomes equal to or greater than a predetermined first slope as the acquisition start point, and determine the acquisition section as a predetermined length based on the acquisition start point. At this time, the predetermined length may be calculated in advance according to the respiration state and the parameter value related to the acquisition length, such as the respiration slope, the respiratory cycle, and the respiratory height, and may be stored in the memory of the MRI system 1 or an external device.

As another example, the MRI system 1 may determine the acquisition section using the respiration state through various methods, such as an interval in which the respiration slope is within the predetermined range, as the acquisition section of the MR data.

On the other hand, the information provided on the user interface may be different depending on whether or not the parameter values related to the protocol are set in advance.

For example, when the parameter value of the protocol is set in advance, the MRI system 1 may display the user interface implemented on the display apparatus to provide the acquisition section automatically determined according to the respiration condition and the acquisition section according to the predetermined protocol.

The MRI system 1 not only provides respiration information, but also automatically acquires an acquisition section according to an acquisition section and a respiration status according to a predetermined protocol, thereby allowing the user to select a desired acquisition section more variously. When any one of the acquisition section according to the predetermined protocol and the automatically determined acquisition section according to the respiration status is selected, the MRI system 1 sets the protocol according to the preset protocol or according to the automatically determined acquisition section, and medical MR data can be collected according to the set protocol.

In addition, when any one of the acquisition section and the acquisition section automatically determined according to the respiration status is changed according to the user's operation command, the MRI system 1 may set or modify the protocol according to the changed acquisition section. Then, the MRI system 1 may collect medical MR data according to the set or modified protocol, and may provide clearer medical image data.

Meanwhile, the disclosed embodiments may be implemented in the form of a computer-readable recording medium for storing instructions and data executable by a computer. The instructions may be stored in the form of program code, and when executed by the processor, may generate a predetermined program module to perform a predetermined operation. In addition, the instructions, when executed by a processor, may perform certain operations of the disclosed embodiments.

The embodiments disclosed with reference to the accompanying drawings have been described above. It will be understood by those skilled in the art that the present invention may be practiced in other forms than the disclosed embodiments without departing from the spirit or essential characteristics of the present invention. The disclosed embodiments are illustrative and should not be construed as limiting.

## Claims

1. A workstation comprising:
a display apparatus; and
a controller configured to control the display apparatus to display respiration information of an object, an acquisition section of MR data related to the object, and a user interface configured to change the displayed acquisition section according to a user's operation command.

2. The workstation according to claim 1, wherein
the controller modifies a protocol to correspond to the changed acquisition section when the displayed acquisition section is changed according to the user's operation command, and acquires MR data according to the modified protocol.

3. The workstation according to claim 1, wherein
the controller controls the display apparatus to display the user interface implemented so that the displayed acquisition section and the changed acquisition section are displayed together when the displayed acquisition section is changed according to the user's operation command.

4. The workstation according to claim 1, wherein
the controller controls the display apparatus to display the user interface implemented so that the displayed acquisition section and the changed acquisition section are displayed with different visual representations when the displayed acquisition section is changed according to the user's operation command.

5. The workstation according to claim 4, wherein
the controller controls the display apparatus to display the user interface implemented so that the displayed acquisition section and at least one of a color and a transparency of the changed acquisition section are set differently.

6. The workstation according to claim 1, wherein
the controller identifies a change in an acquisition time and an acquisition length constituting the acquisition section when the displayed acquisition section is changed, and changes a parameter value related to a protocol according to the identification result.

7. The workstation according to claim 1, wherein
the controller controls the display apparatus to display the user interface implemented to provide a message as to whether to modify a protocol according to the changed acquisition section when the displayed acquisition section is changed according to the user's operation command.

8. The workstation according to claim 1, wherein
the controller controls the display apparatus to display the user interface implemented to display the acquisition section using at least one of an arrow, a line, a symbol, and a polygonal shape.

9. A workstation comprising:
a display apparatus; and
a controller configured to control the display apparatus to display an acquisition section of MR data determined based on respiration information of an object and a respiration state according to the respiration information of the object, and control the display apparatus to display a user interface implemented to receive confirmation of whether to set a protocol to correspond to the displayed acquisition section.

10. The workstation according to claim 9, wherein
the controller sets the protocol to correspond to the acquisition section when receiving a confirmation command from the user, and acquires MR data according to the set protocol.

11. The workstation according to claim 9, wherein
the controller controls the display apparatus to display the user interface implemented to provide at least one of the acquisition section according to a predetermined protocol according to whether the protocol is pre-set and the acquisition section determined by the respiration state.

12. The workstation according to claim 11, wherein
the controller controls the display apparatus to display the user interface implemented so that the acquisition section according to the predetermined protocol and at least one of a color and a transparency automatically determined according to the respiration state are set differently.

13. The workstation according to claim 9, wherein
the controller automatically determines the acquisition section using a respiration slope determined based on the respiration information and controls the display apparatus to display the user interface implemented to provide a message as to whether to set the protocol to correspond to the automatically determined acquisition section.

14. The workstation according to claim 11, wherein
the controller modifies or sets the protocol to correspond to any one of the acquisition section according to the predetermined protocol and the acquisition section determined according to the respiration state.

15. A controlling method of a medical imaging apparatus comprising;
displaying respiration information of an object and an acquisition section of MR data related to the object;
displaying a user interface implemented on a display apparatus to change the displayed acquisition section according to a user's operation command; and
modifying a protocol to correspond to the changed acquisition section when the displayed acquisition section is changed according to an operation command of the user, and acquiring MR data according to the modified protocol.
